# EUROPÄISCHE PATENTANMELDUNG

(11) **EP 2 476 456 A1**
(43) Veröffentlichungstag der Anmeldung: **18.07.2012**
(21) Anmeldenummer: 11194027.6
(22) Anmeldetag: 16.12.2011
(51) Int. Cl.: A61N 1/05

(54) **Selbstauflösendes Elektroden- oder Sondenimplantat**

(30) Priorität: 13.01.2011 US 201161432213 P
(71) Anmelder: BIOTRONIK SE & Co. KG, 12359 Berlin (DE)
(72) Erfinder: Hauer, Marc, 8050 Zürich (CH); Dörr, Thomas, 12437 Berlin (DE)
(74) Vertreter: Lindner-Vogt, Karin L.

(57) **Zusammenfassung**

Die vorliegende Erfindung betrifft dauerimplantierbare Elektrodenstrukturen oder Sonden, wie sie insbesondere bei Herzschrittmachern, ICDs, CRT-Ds oder Neurostimulatoren verwendet werden. Erfindungsgemäß weisen derartige Elektrodenstrukturen oder Sonden mindestens ein Regelelement auf, dessen physikalisch-chemischer Zustand mittels externer Anregung dahingehend gezielt manipulierbar ist, dass in diesem Bereich eine lokale Degradation oder Auflösung eines Teils des Implantats oder des gesamten Implantates einsetzt. Durch diese Teilauflösung wird beispielsweise erreicht, dass sich die Elektrodenstruktur oder zumindest ein Teil davon dahingehend verändert, dass sich die Bedingungen für eine Explantation verbessern, bzw. im Körper verbleibende Teile einer implantierten Elektrodenstruktur funktionell deaktiviert werden.

## Beschreibung

Die Erfindung liegt auf dem Gebiet der Humanmedizin. Sie betrifft Elektrodenanordnungen oder Sonden, die als Implantat in den menschlichen Körper eingesetzt werden.

Derartige Implantate haben in vielfältiger Ausführungsform Anwendung in der modernen Medizintechnik gefunden und werden beispielsweise bei Herzschrittmachern, ICD's, CRT-Ds oder Neurostimulatoren eingesetzt.

Es ist bekannt, dass bei dauerimplantierbaren Elektrodenleitungen oder Sonden aufgrund der längeren Verweilzeiten im menschlichen Körper Teile des Implantats mit körpereigenem Gewebe zumindest partiell verwachsen können. Um diesen unerwünschten Effekt zu reduzieren, werden Elektroden oder Sonden derzeit mit entsprechend hemmenden Oberflächenbeschichtungen versehen. So ist bekannt, dass eine Steroid-Elution einem Einwachsen von Elektroden oder Sonden entgegenwirkt. Außerdem wird für verbesserte Explantationseigenschaften angestrebt, eine möglichst durchgängig glatte Oberfläche des Implantats zu realisieren, indem beispielsweise die Schockwendel einer ICD-Elektrode in den Elektrodenkörper eingebettet wird. Als weitere Maßnahme wird häufig ein isodiametrisches oder sich nach distal verjüngendes Implantatsdesign gewählt.

Trotz dieser Maßnahmen ist ein Einwachsen von Elektrodenleitungen oder Sonden im körpereigenen Gewebe langfristig nicht vollständig zu verhindern. Dies kann zu Schwierigkeiten bei Explantationen führen, da nicht ausgeschlossen werden kann, dass dabei Teile des Gewebes zerstört werden. Eine solche Gewebsschädigung kann je nach betroffener Körperregion zu erheblichen Komplikationen führen.

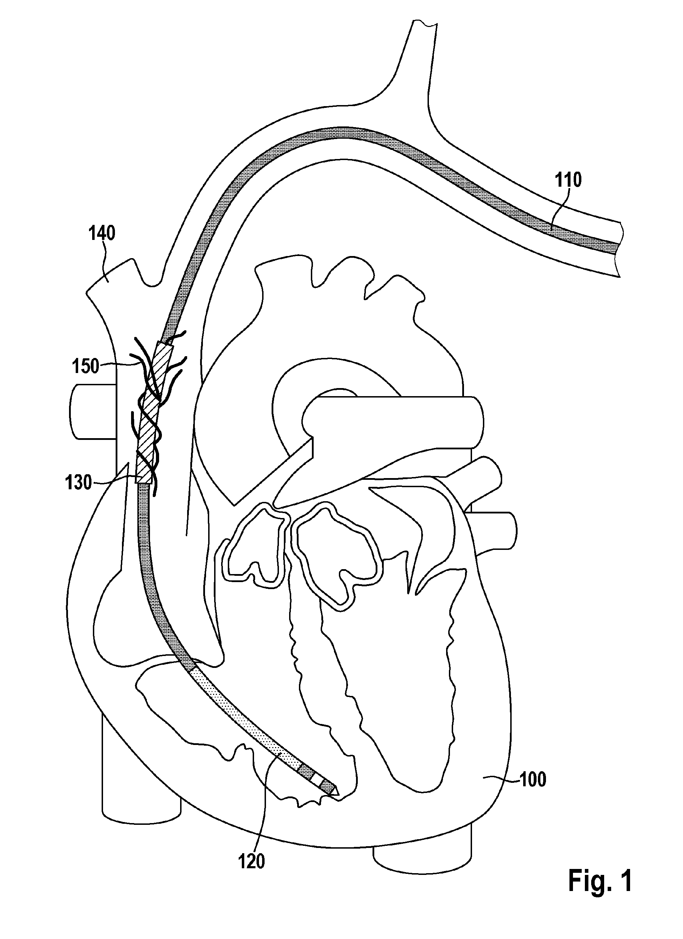

Eine besonders kritische Situation ist in der Figur 1 dargestellt. Hier ist als Beispiel in das menschliche Herz 100 eine endokardiale ICD-Elektrode 110 implantiert. Diese besitzt eine distale Schockwendel 120, die im rechten Ventrikel des Herzens liegt und eine proximale Schockwendel 130, die ausgangs des rechten Vorhofs zum größten Teil in der Vena Cava Superior 140 liegt. Aufgrund der Wendelstruktur erfolgt trotz Form- und Oberflächenoptimierung bei den meisten dieser Elektroden ein Verwachsen 150 der Schockwendel mit den Gefäßwänden. Im Falle einer Sondenextraktion stellen derartige Verwachsungen mit der Gefäßwand der Vena Cava das größte Risiko dar, da hier sehr schnell eine Gefäßruptur der Vena Cava auftreten kann Hauser et.al., Deaths and serious injuries associated with ICD and pacemaker lead extraction; European Heart Journal (2009), 30 (Abstract Supplement), 277*.*

Andererseits ist ein Verbleib von stillgelegten Elektroden oder Sonden im Körper aus anderen Gesichtspunkten nicht unproblematisch. Werden derartige Implantate im Körper belassen, so können Komplikationen wie Gefäßverschlüsse, Interaktionen von stillgelegter Elektrode mit aktiven Elektroden oder aber eine Kontraindikation zum MRT die Folge sein.

Der vorliegenden Erfindung liegt daher die Aufgabe zugrunde, dauerimplantierbare Elektrodenleitungen oder Sonden zu entwickeln, die so ausgebildet sind, dass ihre Explantation wesentlich vereinfacht durchgeführt werden kann, bzw. bei einem Verbleib im Körper das Auftreten nachfolgender Komplikationen nennenswert reduziert wird.

Diese Aufgabe wird bei einer dauerimplantierbaren Elektrode oder Sonde mit den Merkmalen des Oberbegriffs des Anspruchs 1 gelöst durch die kennzeichnenden Merkmale des Anspruchs 1. Weitere Merkmale vorteilhafter Ausführungsformen der Erfindung sind Gegenstand der Unteransprüche.

Erfindungsgemäß weist eine implantierbare Elektrodenleitung oder Sonde mindestens ein Regelelement auf, dessen physikalisch-chemischer Zustand mittels externer Anregung dahingehend manipulierbar ist, dass in diesem Bereich eine lokale Degradation oder Auflösung eines Teils des Implantats oder des gesamten Implantats - auch in Folge der lokalen Degradation - einsetzt. Dieser Prozess führt dann beispielsweise dazu, dass es zu einer Trennung zwischen vorhandenen Verwachsungen eines Elektrodenimplantats mit Körpergewebe kommt, indem sich Oberflächenbereiche der implantierten Elektrode im Bereich der Verwachsungen zumindest partiell auflösen. Alternativ kann durch eine stimulierte lokale Auflösung eines Bereichs einer implantierten Elektrode oder Sonde erreicht werden, dass sich Elektrodenstrukturen an bevorzugten Stellen verjüngen und damit die Explantation wesentlich erleichtert wird. In gleicher Weise können gezielt Sollbruchstellen erzeugt werden, die es dann ermöglichen, wesentliche Teile eines Implantats zu entfernen, ohne dass zu große Beschädigungen des Gewebes entstehen.

Platzierung und Anzahl der mittels externer Anregung von Regelelementen zu einer Degradation bzw. Auflösung stimulierbaren Bereiche können je nach Art und Einsatzfeld des Implantats in weiten Grenzen variiert werden. Somit können einerseits entweder nur vordefinierte Stellen oder Teile der vorliegenden Elektrodenstruktur oder Sonde zur Auflösung angeregt werden und andere Bereiche verbleiben unverändert, andererseits kann aber beispielsweise auch erreicht werden, dass der mittels Stimulation bewirkte Auflösungsprozess eine implantierte Elektrode oder Sonde vollständig erfasst, so dass deren Volumen sich insgesamt verkleinert oder die Elektrode oder Sonde sogar komplett aufgelöst wird.

Die Erfindung wird im Folgenden anhand bevorzugter Ausführungsbeispiele unter Bezugnahme auf die Zeichnungen und die darin aufgeführten Bezugszeichen näher beschrieben.

Es zeigt:
- Fig. 1: eine schematische Darstellung einer in das menschliche Herz implantierten endokardialen ICD-Elektrode mit lokaler Gewebeverwachsung
- Fig. 2: eine schematische Darstellung eines Elektrodenimplantats mit lokaler Gewebeverwachsung und Explantation der Elektrode nach stimulierter teilweiser Auflösung einer äußeren Schicht
- Fig. 3: ein erstes Ausführungsbeispiel einer durch externe Anregung degradierbaren Außenschicht einer implantierbaren Elektrode oder Sonde
- Fig. 4: ein zweites Ausführungsbeispiel einer durch externe Anregung degradierbaren Außenschicht einer implantierbaren Elektrode oder Sonde
- Fig. 5: ein drittes Ausführungsbeispiel einer durch externe Anregung degradierbaren Außenschicht einer implantierbaren Elektrode oder Sonde
- Fig. 6: ein alternatives Ausführungsbeispiel für ein mittels externer Stimulation partiell auflösbares Elektrodenimplantat
- Fig. 7: eine schematische Darstellung der extern angeregten Auflösung eines Teils des Elektrodenimplantats nach Fig. 6
- Fig. 8: eine schematische Darstellung der Extraktion der inneren Elektrodenstruktur nach Auflösung eines Teils des Elektrodenimplantats nach Fig. 6
- Fig. 9: separierte Teilkomponenten des Elektrodenimplantats nach Fig. 6

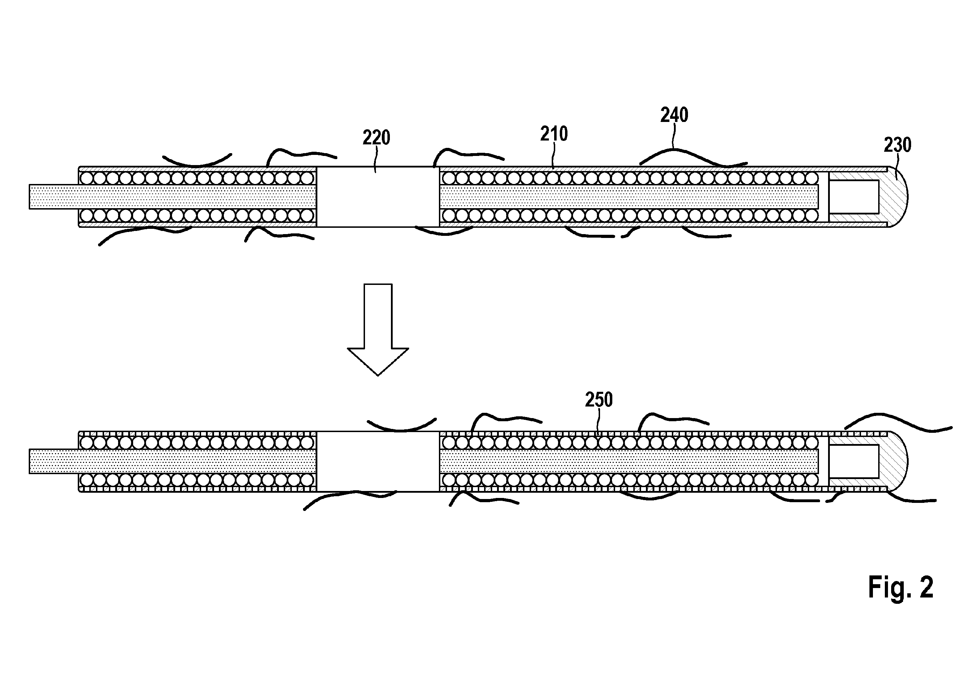

In der Figur 2 ist der distale Teil einer Elektrodenanordnung mit äußerer Isolation 210 dargestellt. Dieser äußere Bereich besteht üblicherweise aus einem Material mit möglichst hoher Körperverträglichkeit (Biokompatibilität). Unter Biokompatibilität wird die Fähigkeit eines Werkstoffes verstanden, in einer spezifischen medizinischen Anwendung eine angemessene Gewebereaktion hervorzurufen. Diese beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Die in Figur 2 dargestellte Elektrodenanordnung weist zwei Stimulationspole auf (Ring 220 und Tip 230). Die Elektrodenleitung ist zu einem großen Teil von körpereigenem Gewebe 240 umwachsen und kann daher nicht durch einfaches Herausziehen explantiert werden. Erfindungsgemäß weist in diesem Ausführungsbeispiel die äußere Isolationsschicht 210 Bereiche 250 auf, in denen mittels externer Anregung von Regelelementen ein Degradationsprozess der Isolationsschicht ausgelöst werden kann. Durch die teilweise oder völlige Auflösung dieser Bereiche 250 der Isolationsschicht 210 wird die Haftung zwischen der Oberfläche der Isolationsschicht 210 der implantierten Elektrode und dem umgebenden körpereigenem Gewebe 240 erheblich reduziert und die Gefahr von Gewebsschädigungen bei einer Explantation durch Herausziehen des Implantats ist deutlich verringert.

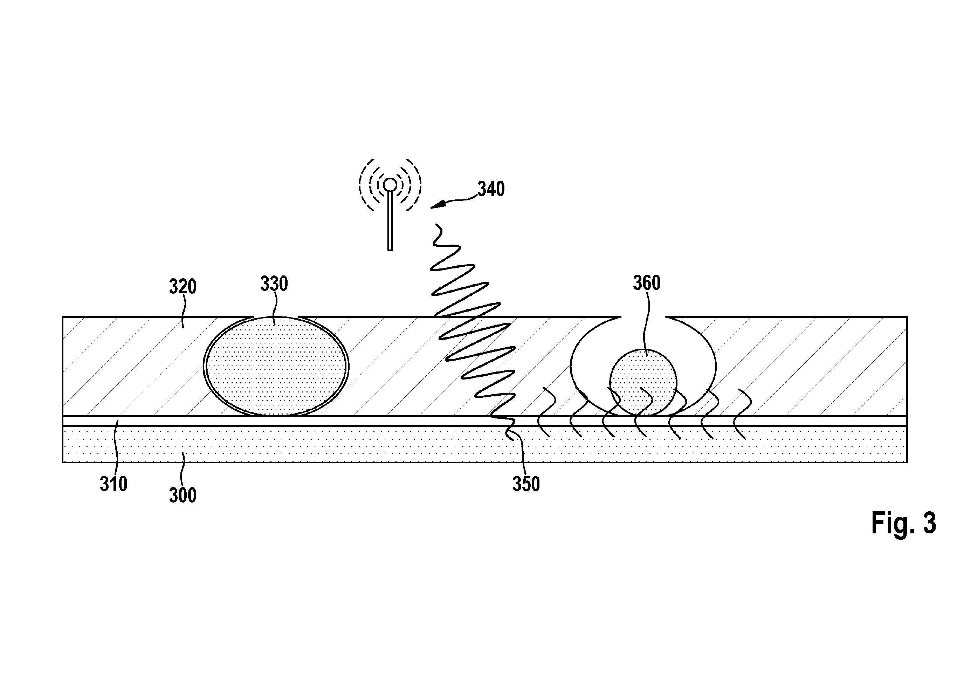

Die Figur 3 zeigt schematisch ein erstes Ausführungsbeispiel für eine implantierbare Elektrode oder Sonde, die gemäß Figur 2 eine äußere Beschichtung aufweist, bei der erfindungsgemäß Bereiche vorhanden sind, die durch externe Anregung degradierbar sind. Auf der inneren Elektrodenstruktur 300 ist, z.B. mittels eines Haftvermittlers 310, zumindest abschnittsweise eine Isolationsschicht 320 aufgebracht. Die Isolationsschicht 320 ist nicht biokorrodierbar und kann beispielsweise Poly-L-Lactid enthalten oder daraus bestehen oder einen weiteren Vertreter der Polyester wie PDLLA, PLGA, P3HB, P4HB oder Gemische oder Copolymere daraus. Alternativ oder zusätzlich kann die Schutzschicht Parylen aufweisen (Parylen C oder andere Derivate), bevorzugt als Parylen mit sog. "pin holes". Daneben oder stattdessen kann die Schutzschicht Cellulose aufweisen, bevorzugt als Film, wie beispielsweise Nitrocellulose, Methylcellulose, Carboxymethylcellulose. Die Schutzschicht kann auch Polyvinylalkohole aufweisen, wobei durch Wahl der Molmasse und des Deacetylierungsgrades eine Filmbildung optimiert werden kann. Polyvinylalkohol ist ein kristallines Polymer, das aufgrund seines Herstellungsverfahrens geringfügig verzweigt ist. Polyvinylacetat wird aus Vinylacetat hergestellt. Das Polyvinylacetat wird durch Umsatz mit Basen zum Polyvinylalkohol hydroliesiert. Die Schmelz- und Glasübergangstemperatur hängt außer vom Hydrolysegrad und der Molmasse auch von der Verteilung der Acetylgruppen (statistisch oder in Blöcken) der Taktizität und dem Wassergehalt des Polymers ab. Geeignet sind Polyvinylalkohole die hohe bis mittlere Hydrolysierungsgrade aufweisen und Polymerisationsgrade bis 2000 besitzen. Die aus Polyvinylalkohol hergestellten Filme sind reißfest und zähelastisch. Sie sind öl- und hitzebeständig. Als Weichmacher können Polyalkohole, wie z. B. Glycerin und Ethylenglycol, verwendet werden.

Die Isolationsschicht 320 weist Regelelemente in Form von Perforationsstellen 330 auf, die mit einem Hydrogel aufgefüllt sind. Ein Hydrogel ist ein Wasser enthaltendes, aber wasserunlösliches Polymer, dessen Moleküle chemisch, z. B. durch kovalente oder ionische Bindungen oder physikalisch, z. B. durch Verschlaufen der Polymerketten, zu einem dreidimensionalen Netzwerk verknüpft sind. Erfindungsgemäße Hydrogele sind in der Lage als Reaktion auf eine externe Anregung ihr Volumen zu verändern, in dem sie ein veränderbares Quellvermögen aufweisen und damit regel- und/oder steuerbar unterschiedlich viel Wasser pro mmol Hydrogel-Polymer aufnehmen können.

Diese Hydrogele können beispielsweise hergestellt werden durch Umsetzung ethylenisch ungesättigter Monomere und Polymere die ionisierbare Gruppen tragen mit Vernetzern und Polymerisationskatalysatoren. Alternativ dazu können geeignete Hydrogele hergestellt werden durch Kondensationsreaktionen mit difunktionellen und mehrfunktionellen Monomeren. Dem Fachmann sind geeignete Monomere und Polymere sowie Verfahren zu deren Herstellung bekannt. Ebenso sind dem Fachmann Methoden und Verfahren zur Herstellung von geeigneten Hydrogelen mittels solcher Mono- oder/und Polymere bekannt. Bevorzugte Hydrogele enthalten ein Polymer auf Basis von Acrylamid, Methacrylamid, Dimethylaminoethylmethacrylate oder einem Derivat von Acrylamid, Methacrylamid oder Dimethylaminoethylmethacrylate. Andere bevorzugte Hydrogele enthalten ein Polymer auf Basis von poly N-Isopropylacrylamide und/oder Poly-N-isopropylacrylamide-co-allylamin und/oder Poly-N-Isopropylamid (PNIPAM) oder Mischungen daraus mit Poly(p-dioxanon) als Hartsegment.

Unter dem Begriff "Quellvermögen" im Sinne der Erfindung wird die Eigenschaft des Hydrogels verstanden, eine bestimmte Wassermenge pro mmol Hydrogel-Polymer aufzunehmen. Ein verringertes Quellvermögen führt zu einer Verringerung des Volumens des Hydrogels und damit zu einer Formänderung des Hydrogels. Geeignete Methoden und Messverfahren zur Bestimmung des Quellvermögens sind dem Fachmann bekannt, insbesondere eignen sich solche Messverfahren, wie sie sich im Bereich der Galenik bereits vielfach bewährt haben.

Bevorzugt werden Hydrogele eingesetzt, deren Quellvermögen temperaturabhängig ist. Besonders bevorzugte Hydrogele weisen bei steigenden Temperaturen ein verringertes Quellvermögen auf. Solche Hydrogele können sich dadurch auszeichnen, dass sich ihr Quellvermögen bei einer Temperaturerhöhung von 10K mindestens um 30% verringert, bevorzugt um bis zu 50%, besonders bevorzugt um 30% bis 50%.

Die Temperaturabhängigkeit des Hydrogels ist dabei bevorzugt so eingestellt, dass es bezüglich der Quelleigenschaften einen ausgeprägten Hystereseeffekt gibt, so dass das Quellvermögen auch bei Rückkehr zur Ausgangstemperatur von 37°C reduziert bleibt.

Wie in Figur 3 schematisch dargestellt, kann in diesem Ausführungsbeispiel eine temporäre Erwärmung der Isolationsschicht 320 und damit auch des Hydrogels in den Perforationsstellen 330 durch externe Einprägung von Hochfrequenz-Energie mittels eines Hochfrequenzsenders 340 ausgelöst werden. Vorzugsweise ist die Wellenlänge des Hochfrequenz-Senders 340 auf die Antennengeometrie 350 der Elektrodenstruktur abgestimmt, so dass eine effektive Erwärmung möglich ist. Durch die induzierte Erwärmung der Elektrodenstruktur wird auch das Hydrogel erwärmt und dessen Quelleigenschaften so weit reduziert, dass das Hydrogel eine Formänderung durchläuft, hin zu einem reduzierten Hydrogelvolumen 360, so dass die Perforationsstelle 330 in der Isolationsschicht 320 weitgehend freigelegt ist. Bei entsprechender Anzahl derartiger Perforationsstellen 330 wird durch die Volumenabnahme des Hydrogels die Haftung zwischen der Oberfläche der Isolationsschicht 320 und dem umgebenden körpereigenem Gewebe insgesamt erheblich reduziert.

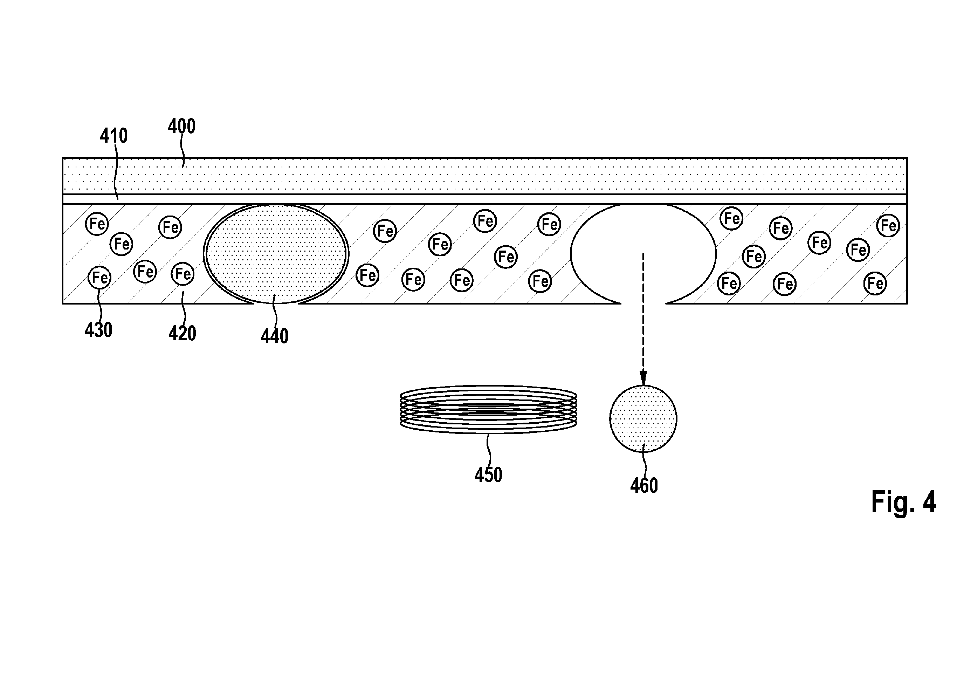

In Figur 4 ist eine alternative Ausführungsform einer durch externe Anregung degradierbaren Außenschicht einer implantierbaren Elektrode oder Sonde dargestellt. Auch hier ist auf der Elektrodenstruktur 400 des Implantates 400 mittels Haftvermittler 410 eine Isolationsschicht 420 mit Perforationsstellen 440 aufgebracht. Diese Isolationsschicht 420 beinhaltet magnetische Nanopartikel 430. Die Perforationsbereiche 440 sind auch hier mit einem Hydrogel verschlossen. Bei dieser Ausführungsform kommt ein Hydrogel mit einem diskontinuierlichen Quellvermögen zum Einsatz, welches oberhalb einer bestimmten Temperatur (z. B. 45°-50°C) schlagartig zu einem reduzierten Hydrogel 460 kollabiert und dabei aus der Perforationsstelle der Schutzschicht "herausfällt". Die Erwärmung der Isolationsschicht 420 und damit des Hydrogels erfolgt hier durch ein wechselndes Magnetfeld, das mittels eines externen magnetischen Wechselfeldgenerators 450 eingeprägt wird. Dabei werden die magnetischen Nanopartikel 430 in Schwingung versetzt und somit die Isolationsschicht 420 und das Hydrogel in den Perforationsstellen 440 erwärmt.

Bei den Ausführungsformen gemäß Fig. 3 und 4 kann nach erfolgter externer Anregung und damit Öffnung der Perforationsstellen 330, 440 auch Körpermedium die innere Elektrodenstruktur 300, 400 erreichen, sofern die Haftbrücke 310, 410 entsprechend dafür durchlässig ausgelegt worden ist. Wird dabei zusätzlich für die innere Elektrodenstruktur 300, 400 ein biokorrodierbarer metallischer Werkstoff gewählt, so erfasst der durch externe Anregung ausgelöste Degradationsprozess in Folge durch Kontakt mit Körpermedium auch die innere Elektrodenstruktur 300, 400 in den Bereichen, in denen die Isolationsschicht 320, 420 Perforationsstellen 330, 440 aufweist. Als Körpermedium im Sinne der Erfindung werden alle im menschlichen Körper natürlich vorhandenen oder auch durch Aufnahme in den Körper zusätzlich eingebrachten Medien verstanden. Dazu gehören Flüssigkeiten, die Wasser enthalten, wie beispielsweise Blut, Lymphflüssigkeit, Speichel und andere Stoffe, insbesondere Ionen, aber auch Gase, wie z.B. Kohlendioxid, Wasserstoff, Sauerstoff und Stickstoff.

Im Bereich biokorrodierbarer metallischer Werkstoffe wird Magnesium oder Reineisen verwendet, sowie biokorrodierbare Basislegierungen der Elemente Magnesium, Eisen, Zink, Molybdän, Wolfram oder Aluminium. Zusätzlich sind Legierungen aus den genannten Basiselementen mit zusätzlich einem Element aus der Gruppe der Alkalimetalle oder Erdalkalimetalle bekannt. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Kalzium, Lithium, Aluminium, Zink und Eisen sein. Besonders geeignet ist der Einsatz einer biokorrodierbaren Magnesiumlegierung mit einem Anteil von Magnesium >90%, Yttrium 3,7 - 5,5%, Seltenerdmetallen 1,5 - 4,4% und Rest <1%.

Durch Kombination von lokal mittels externer Anregung von Regelelementen degradierbarer Beschichtung und einer aus biokorrodierbarem Werkstoff bestehenden Elektrodenstruktur können durch entsprechende Anordnung der Degradationsstellen gezielt vordefinierte Teile einer implantierten Elektrodenstruktur in einen Korrosionsprozess einbezogen werden - oder auch die Elektrodenstruktur insgesamt.

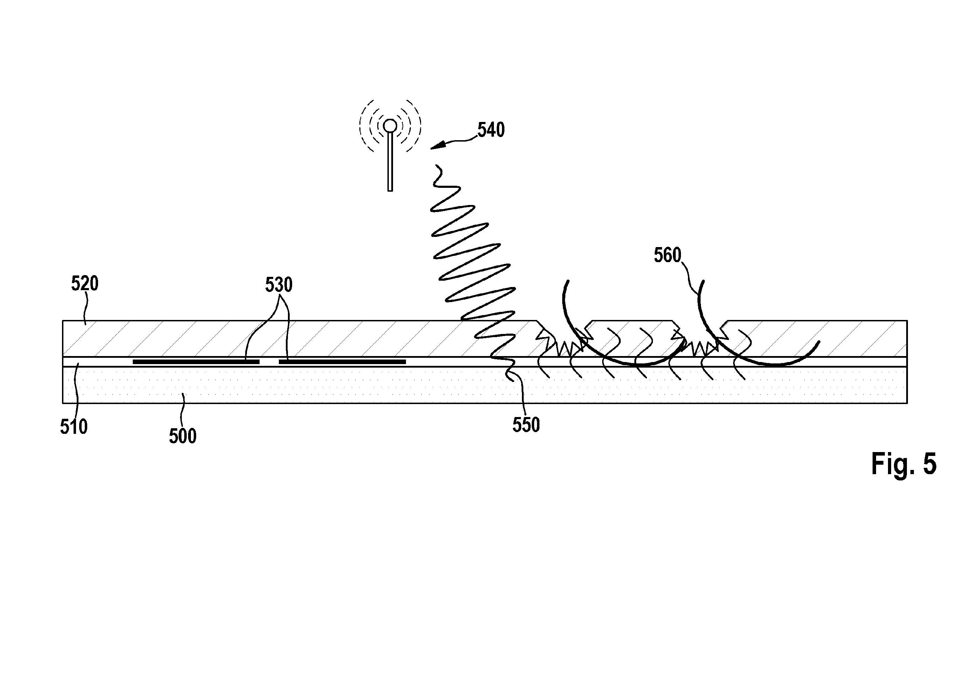

In Figur 5 ist eine weitere Ausführungsform der Erfindung schematisch dargestellt, bei nach externer Anregung von Regelelementen eine Degradation lokaler Bereiche einer Isolationsschicht einsetzt und damit durch entstehende Porenbildung ein Kontakt von Körperflüssigkeit mit inneren Elektrodenstrukturen des Implantats bewirkt wird. Auch in diesem Ausführungsbeispiel ist auf der inneren Elektrodenstruktur 500 des Implantates mittels Haftvermittler 510 eine Isolationsschicht 520 aufgebracht. Der Haftvermittler 510 beinhaltet in diesem Ausführungsbeispiel als Regelelemente zusätzlich Formkörper 530, bestehend aus einem biokompatiblen, vorzugsweise biodegradierbaren Formgedächtnis-Polymer. Formgedächtnis-Polymere sind Kunststoffe, die einen so genannten "shape memory"-Effekt aufweisen. Unter einem "shape memory"-Effekt wird dabei verstanden, dass ein Formgedächtnis-Polymer aus einer ursprünglichen Form in eine andere Form stabil überführt werden kann, in der es dann solange verbleibt, bis das Formgedächtnis-Polymer, z.B. als Reaktion auf eine externe Anregung, zu einer vorherigen oder der ursprünglichen Form zurückkehrt.

In der dargestellten Ausführungsform ist die Ursprungsform des Formgedächtnis-Polymer eine gebogene Form und die planare Form des Formkörpers 530 stellt den "deformierten Zustand" (eingestellt bei 125°C) des Formgedächtnis-Polymer dar. Durch eine mittels eines externen Hochfrequenzsenders 540 eingeprägte Hochfrequenz-Energie wird die Elektrodenstruktur 500 und somit auch die benachbarten Formkörper 530 für mindestens 10 Sekunden auf 45°C erwärmt. Dabei nimmt das Formgedächtnis-Polymer der Formkörper 530 seine ursprüngliche, gebogene Form wieder an. Mittels der nun in der Form veränderten Formkörper 560 wird auch die Isolationsschicht 520 zum Teil perforiert und Körperflüssigkeit erreicht in diesem Bereich die innere Elektrodenstruktur 500.

Alternativ kann hier die Formveränderung des Formgedächtnis-Polymer durch Licht ausgelöst werden. Eine denkbare Anwendung besteht darin, dass beispielsweise mittels Lichtleitkatheter durch Einstrahlung von UV-Licht mit einer Wellenlänge kleiner 260 Nanometer die Formänderung gestartet wird.

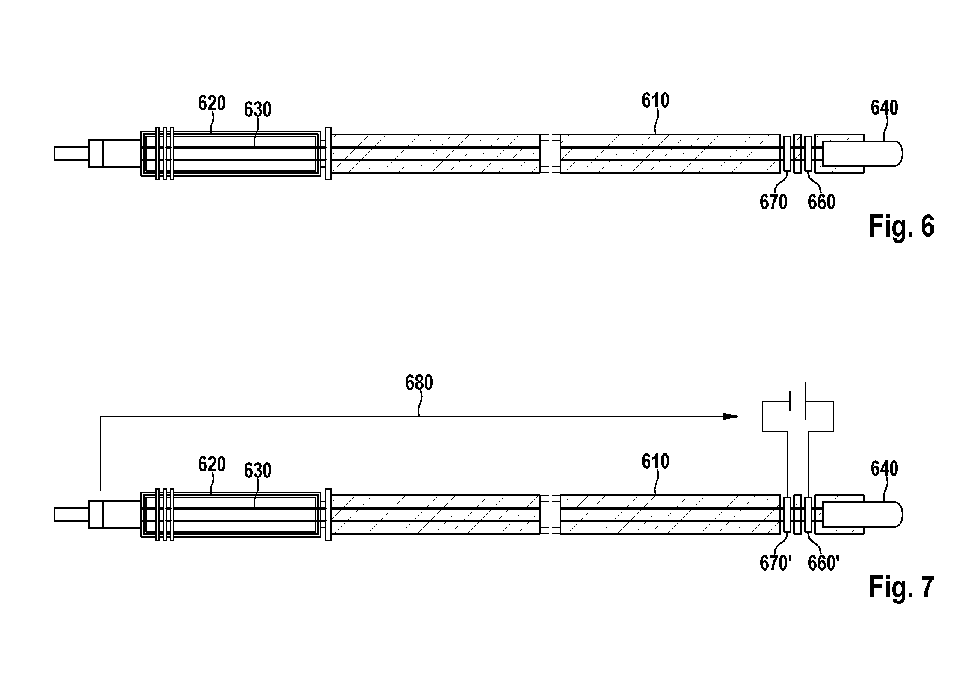

Die Figur 6 zeigt ein alternatives Ausführungsbeispiel für eine mittels externer Stimulation partiell auflösbare Elektrodenstruktur. Das Implantat besteht aus einem äußeren Isolatorschlauch 610, einem bipolaren Elektrodenstecker 620, metallischen Elektrodenzuleitungen 630 und mindestens einem Elektrodenpol 640. In der dargestellten Anordnung sind die Zuleitungen und der Elektrodenkopf mit metallischen Fixatoren 660, 670 im distalen Bereich mit dem äußeren Isolator fest verbunden, so dass die Elektrodenstruktur eine stabile Einheit bildet.

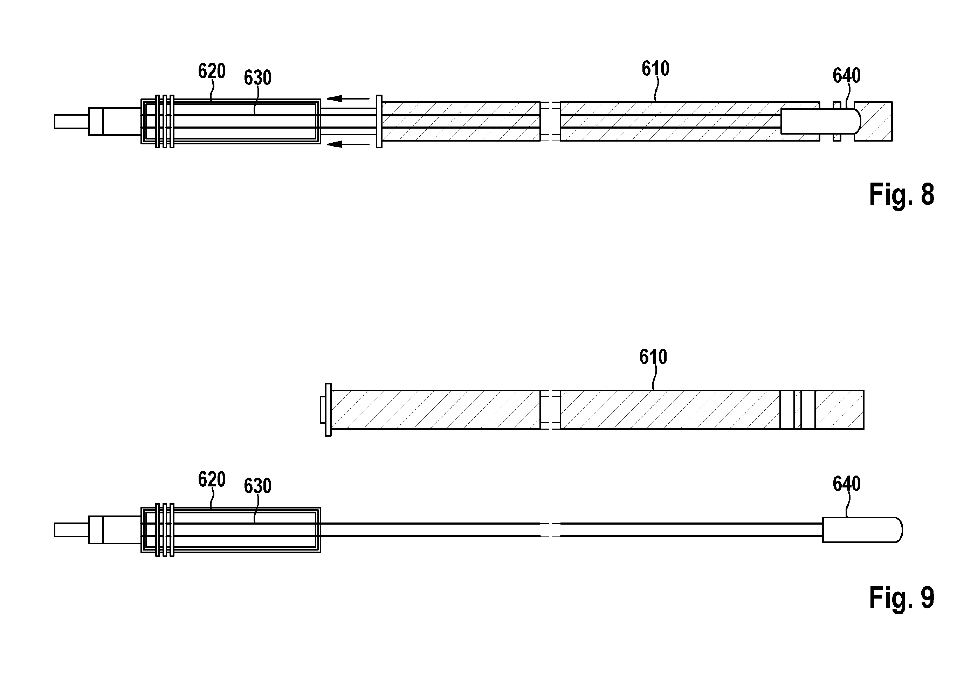

Vor einer Explantation dieser Elektrodenstruktur kann bei dieser Ausführungsform nach Figur 6 erfindungsgemäß gezielt der Bereich der Fixatoren 660, 670 als Regelement extern angeregt werden. Dies ist dargestellt in Figur 8. Durch Anlegen einer entsprechenden Spannung 680 über den Elektrodenstecker 620 wird durch die Potentialdifferenz eine Korrosion der Fixatoren eingeleitet (660', 670'). Je nach entsprechender Einwirkzeit der externen Anregung werden dadurch die Fixatoren 660, 670 geschwächt, oder, wie in Figur 8 dargestellt, sogar vollständig aufgelöst und die innere Elektrodenstruktur kann gegenüber der äußeren Isolation 610 herausgezogen werden.

Wie in Figur 9 dargestellt, zerfällt das zuvor einheitliche Elektrodenimplantat mittels externer Anregung der Degradation eines lokalen Bereichs 660, 670 in zwei separierbare Komponenten. Alle leitfähigen Bestandteile, also Elektrodenstecker 620, metallische Elektrodenzuleitungen 630 und Elektrodenpol 640 lassen sich extrahieren. Die äußere Isolationsschicht 610 kann anschließend auch im Körper verbleiben und als Einführungskanal beispielsweise für eine neue Elektrode dienen.

Ein Bereich, der wie in den Figuren 7 bis 9 skizziert, erfindungsgemäß durch externe Anregung von Regelelementen einer lokalen Korrosion zugeführt werden kann, kann an verschiedenen vordefinierten Stellen einer implantierbaren Elektrodenstruktur oder Sonde vorgesehen werden. Damit können bei Anlegen entsprechender externer Spannung mittels unterschiedlicher Potentialdifferenz gezielt verschiedene Bereiche der Elektrodenstruktur elektrolytisch aufgelöst werden. Je nach Wahl des metallischen Werkstoffs und der Potentialdifferenz kann dabei die Geschwindigkeit des einzelnen Auflösungsprozesses gesteuert werden. Damit kann der Vorgang so eingestellt werde, dass während des Auflösungsvorgangs eine Verträglichkeit für den menschlichen Organismus gewährleistet ist.

Die externe Anregung von Regelelementen als Auslöser für Degradationsvorgänge an vorgegebenen Bereichen implantierter Elektrodenstrukturen oder Sonden kann in alternative Weise auch durch Gabe von Medikamenten, bzw. Einbringen spezieller chemischer Substanzen erfolgen, die eine Auflösung lokaler Bereiche der Elektrodenstruktur beispielsweise durch katalytische Wirkung bewirken. Dazu weist eine implantierte Elektrodenstruktur an den gewünschten Stellen entsprechende Bereiche ausgewählter Materialien auf, die mit den hinzugeführten Substanzen im Sinne einer Degradation in physikalisch/chemische Wechselwirkung treten.

Alle dargestellten Ausführungsbeispiele der Erfindung lassen sich auch kombinieren, so dass beispielsweise auf einer Elektrodenstruktur einerseits Bereiche vorgesehen sein können, die durch externe HF-Anregung degradieren, zusätzlich aber auch Bereiche vorhanden sind, die mittels extern aufgebrachter Potentialdifferenz elektrolytisch korrodieren.

## Patentansprüche

1. Dauerimplantierbare Elektrode oder Sonde, bestehend aus einer metallischen Elektrodenstruktur, die zumindest teilweise von einer äußeren Schutzschicht umgeben ist, welche vorzugsweise aus biokompatiblem Material besteht und isolierende elektrische Eigenschaften aufweist und für Körpermedium undurchlässig ist,
**dadurch gekennzeichnet, dass**
das Elektroden- oder Sondenimplantat mindestens ein Regelelement aufweist, dessen physikalisch-chemischer Zustand mittels einer externen Anregung dahingehend gezielt modifizierbar ist, dass in dem Bereich des Regelelements eine lokale Degradation eines Teils des Elektroden- oder Sondenimplantats oder des gesamten Implantates einsetzt.

2. Elektroden- oder Sondenimplantat nach Anspruch 1, **dadurch gekennzeichnet, dass** mindestens ein Regelelement, beispielsweise durch seine Form und Platzierung, als mechanische Fixierung der Elektrodenstruktur gegenüber der umgebenden Schutzschicht ausgeführt ist, so dass die durch externe Anregung ausgelöste Auflösung im Bereich des Regelelements die Fixierung der Elektrodenstruktur gegenüber der umgebenden Schutzschicht aufhebt.

3. Elektroden- oder Sondenimplantat nach Anspruch 1 oder 2, **dadurch gekennzeichnet, dass** mindestens ein Regelelement als definierter Teil der Elektrodenstruktur ausgebildet ist und die externe Anregung in diesem Bereich eine lokale Degradation der Elektrodenstruktur durch elektrolytische Korrosion auslöst.

4. Elektroden- oder Sondenimplantat nach Anspruch 3, **dadurch gekennzeichnet, dass** die externe Anregung eine mittels Elektrodenleitung an das Regelelement zugeführte elektrische Spannung darstellt.

5. Elektroden- oder Sondenimplantat nach Anspruch 1, **dadurch gekennzeichnet, dass** mindestens ein Regelelement dergestalt in die Schutzschicht integriert ist, dass die mittels externer Anregung lokal ausgelöste Degradation einen Bereich der Schutzschicht betrifft.

6. Elektroden- oder Sondenimplantat nach Anspruch 5, **dadurch gekennzeichnet, dass** die Degradation eine lokale Perforation der Schutzschicht darstellt.

7. Elektroden- oder Sondenimplantat nach Anspruch 5 oder 6, **dadurch gekennzeichnet, dass** die mittels externer Anregung bewirkte Modifizierung des physikalischchemischen Zustands eines Regelelements eine Formänderung darstellt.

8. Elektroden- oder Sondenimplantat nach Anspruch 5, 6 oder 7, **dadurch gekennzeichnet, dass** ein Regelelement ein Hydrogel enthält oder daraus besteht.

9. Elektroden- oder Sondenimplantat nach Anspruch 8, **dadurch gekennzeichnet, dass** das Quellvermögen des Hydrogels temperaturabhängig ist, bevorzugt verringert sich das Quellvermögen bei steigenden Temperaturen.

10. Elektroden- oder Sondenimplantat nach Anspruch 9, **dadurch gekennzeichnet, dass** das Quellvermögen des Hydrogels sich bei einer Temperaturerhöhung um 10K um mindestens 30% verringert.

11. Elektroden- oder Sondenimplantat nach Anspruch 9 oder 10, **dadurch gekennzeichnet, dass** das Quellvermögen des Hydrogels eine Hysterese aufweist.

12. Elektroden- oder Sondenimplantat nach Anspruch 5, 6 oder 7 **dadurch gekennzeichnet, dass** ein Regelelement ein Formgedächtnis-Polymer enthält oder daraus besteht.

13. Elektroden- oder Sondenimplantat nach einem der Ansprüche 5 bis 12, **dadurch gekennzeichnet, dass** die externe Anregung elektromagnetische Strahlung, insbesondere Hochfrequenzstrahlung oder Licht, Ultraschall, ionisierende Strahlung oder ein magnetisches Feld umfasst oder daraus besteht.

14. Elektroden- oder Sondenimplantat nach einem der Ansprüche 5 bis 12, **dadurch gekennzeichnet, dass** die Elektrodenstruktur zumindest teilweise aus biokorrodierbarem Material, vorzugsweise einer Magnesiumlegierung besteht, welches bei Kontakt mit Körpermedium als Folge der Degradation der Schutzschicht zumindest teilweise aufgelöst wird.

15. Elektroden- oder Sondenimplantat nach einem der vorangehenden Ansprüche, **dadurch gekennzeichnet, dass** die externe Anregung auch die Gabe von Medikamenten oder chemischen Substanzen beinhaltet, welche mit einem Regelelement in Wechselwirkung treten, so dass dessen physikalisch-chemischer Zustand modifiziert wird.
